# EUROPÄISCHE PATENTSCHRIFT

(11) **EP 0 874 618 B1**
(45) Veröffentlichungstag und Bekanntmachung des Hinweises auf die Patenterteilung: **12.11.2003**
(21) Anmeldenummer: 96942236.9
(22) Anmeldetag: 31.12.1996
(51) Int. Cl.: A61K 7/48, A61K 7/06

(54) **PRÄPARAT ZUR VERBESSERUNG DES HAARWUCHSES, DER HAUTSTRUKTUR UND/ODER DER NAGELREGENERATION**
PREPARATION FOR IMPROVING HAIR GROWTH, THE SKIN STRUCTURE AND/OR NAIL REGENERATION
PREPARATION POUR STIMULER LA POUSSE DES CHEVEUX, AMELIORER LA STRUCTURE DE LA PEAU ET/OU REGENERER LES ONGLES

(30) Priorität: 17.01.1996 WO PCT/CH96/00024
(43) Veröffentlichungstag der Anmeldung: 04.11.1998
(73) Patentinhaber: Kisters, Friedrich, 8280 Kreuzlingen 4 (CH)
(72) Erfinder: KISTERS, Friedrich, CH-8280 Kreuzlingen 4 (CH); KESSLER, Günther, verstorben (CH); NÖFER, Reinhold, D-87751 Heimertingen (DE)
(74) Vertreter: Groner, Manfred
(86) Internationale Anmeldenummer: CH9600466
(87) Internationale Veröffentlichungsnummer: WO97025972

(56) Entgegenhaltungen:
- WO-A-96/27371
- DE-A- 3 637 992
- DE-A- 4 243 362
- DE-A- 4 243 363
- GB-A- 2 268 871
- US-A- 5 198 465

## Beschreibung

Die vorliegende Erfindung betrifft ein Präparat zur Verbesserung des Haarwuchses, der Hautstruktur und/oder der Nagelregeneration gemäss dem Oberbegriff des Patentanspruchs 1.

Bei dieser Erfindung handelt es sich um ein diäthetisches Präparat zur Unterstützung beziehungsweise Regelung der verschiedenen Energiestoffwechselwege im menschlichen Körper, sowie dem Aufbau von Strukturproteinen. Eine orale Verabreichung des Präparats gemäss der vorliegenden Erfindung wirkt sich daher positiv insbesondere auf Haarwachstum und Haarstruktur aus.

Als Stoffwechsel bezeichnet man die Gesamtheit der chemischen Umsetzungen in einem Organismus, die zur Aufrechterhaltung der Lebensvorgänge notwendig sind. Diese betreffen die Aufnahme, den Ein-, Um- und Abbau wie auch Ausscheidungen von Stoffen zur Erhaltung bzw. zum Aufbau der Körpersubstanz und natürlich auch zur Energiegewinnung.

Von Energie- oder Betriebsstoffwechsel spricht man beim Umsatz der körpereigenen Stoffe zur Gewinnung von Energie: Als Wärmeenergie zur Aufrechterhaltung der Körperwärme, als mechanische Energie zur Arbeitsleistung der Organe und Muskeln, als chemische Energie für die Synthese von arteigenen Proteinen, Fetten, Glykogen, Polynukleotiden, energiereichen Phosphatverbindungen, zur Ausbildung elektrischer Potentiale, für osmotische Arbeit und so weiter. Eng verzahnt mit dem Energiestoffwechsel ist der Bau- oder Erhaltungsstoffwechsel, der der Produktion (Biogenese) notwendiger Zellbestandteile aus einfachen Bausteinen ebenso dient wie dem Abbau höhermolekularer Stoffe zu niedermolekularen Substanzen, die entweder anderweitig im Organismus für Aufbaureaktionen eingesetzt oder ausgeschieden werden sollen.

Im Energie-, nicht aber im Baustoffwechsel können die Energielieferanten wie Kohlehydrate, Eiweiss und Fette einander in gewissem Mass vertreten, da sie letztlich über ähnliche Abbauwege in Energie umgewandelt werden. Von besonderer Bedeutung sind in diesem Zusammenhang die Aminosäuren. Unter Aminosäuren versteht man die 20 am Aufbau von Proteinen beteiligten (proteinogenen) L-Aminocarbonsäuren. Pflanzen sind in der Lage alle Aminosäuren aus einfacheren Vorstufen zu synthetisieren. Menschen und Tiere können dagegen nur die sogenannten nicht-essentiellen Aminosäuren Alanin, Asparagin, Asparaginsäure, Glutamin, Glutaminsäure, Hydroxy-L-Prolin, Prolin, Glycin und Serin selbst aufbauen. Die essentiellen Aminosäuren Isoleucin, Leucin, Lysin, Methionin, Phenylalanin, Ornithin, Threonin, Tryptophan und Valin müssen mit der Nahrung aufgenommen werden. Als semiessentiell gelten Arginin und Histidin, die nur in Wachstumsphasen und bei Mangelerscheinungen von aussen zugeführt werden müssen. Eine Sonderstellung nehmen Cystein und Tyrosin ein, die aus den essentiellen Aminosäuren Methionin und Phenylalanin synthetisiert werden können. Die wichtigste Funktion der Aminosäuren ist die als Bausteine für die Biosynthese von Proteinen, beim Fehlen von essentiellen Aminosäuren in der Nahrung gerät unter anderem die Eiweissynthese ins Stocken und als weitere Folge stellen sich gegebenenfalls lebensbedrohliche Mangelerscheinungen ein.

Der physiologische Auf- und Abbau der Aminosäuren erfolgt über die Abbauprodukte der Glykolyse und alpha-Ketonsäuren, von denen 2-Oxoglutarsäure und Oxalessigsäure Glieder des Citronensäure-Zyklus sind. Über sie ist der Stoffwechsel von Aminosäuren mit dem von Fetten und Kohlehydraten verknüpft. In bezug auf ihre Abbauprodukte unterscheidet man zwischen glucogenen Aminosäuren, die Intermediate des Citronensäure-Zyklus oder Brenztraubensäure liefern, und ketogenen Aminosäuren, die nicht zur Glukosesynthese verwendet werden können, da sie beim Abbau Ketone wie Acetessigsäure ergeben. Da die Aminosäuren auch grosse Bedeutung als Nährstoffe haben, gibt es verschiedene spezifische zelluläre Transportsysteme, die die Aufnahme von Aminosäuren durch die Membran regulieren.

Das im Normalzustand ausgewogene Verhältnis von Aufnahme, Umsowie Abbau und Ausscheidung der verschiedenen Stoffe, vor allem auch beim Aminosäurestoffwechsel, kann nun durch verschiedene Störungen aus dem Gleichgewicht gebracht werden. Als Beispiele seien Diabetes, Lipidosen, Gicht, Hyperthyreose, Rachitis, Phenylketonurie, Infektionen und viele andere Störungen, die ererbt oder erworben sein können, genannt. Nicht zu vergessen sind natürlich auch stress- und umweltbedingte (z.B. durch Toxine verschiedenster Art) sowie durch Medikamente hervorgerufene Störungen.

Als Folge hiervon können häufig Leistungsabfall, Müdigkeit, Muskelatrophie, Nachlassen der Gehirnleistung, Potenzschwäche oder Veränderungen der Blutfett- und Blutzuckerwerte auftreten. Ein markantes äusseres Zeichen für das Vorliegen einer solchen Störung ist der nicht-hormonbedingte, d.h. Testosteron-unabhängige Haarausfall, der sich als sogenanntes "Effluvium" oder aber als "Alopezie" äussern kann, bei der eine Glatzenbildung auftritt, die herdförmig, diffus oder als totale Glatzenbildung in Erscheinung tritt. Hinsichtlich der Prognose unterscheidet man zwischen reversibler und irreversibler Alopezie, wobei sich die irreversible Alopezie durch eine Zerstörung beziehungsweise Fehlen der Haaranlagen auszeichnet.

Das menschliche Haar durchläuft einen Lebenszyklus, der drei Phasen, d.h. die anagene oder Wachstumsphase, die katagene oder Übergangsphase sowie die telogene oder Ruhephase umfasst. Unter normalen Bedingungen befinden sich 80 bis 85 % der Haare in der Anaphase und 15 bis 20 % in der Übergangs- beziehungsweise Ruhephase. Die anagene Phase ist die Periode des aktiven Haarwachstums und dauert ca. 3 bis 5 Jahre. Die katagene Phase ist eine Übergangsphase zwischen aktivem Wachstum und Ruhephase und dauert etwa 1 bis 2 Wochen. Die sich anschliessende telogene Phase dauert 3 bis 4 Monate. Hier stoppt das Wachstum und das Haar wird gegebenenfalls abgestossen, wonach neues Haarwachstum beginnt. Tritt nun bei einem Patienten vermehrter Haarverlust auf, so kann dies daran liegen, dass ein grösserer Teil als die obengenannten 15 bis 20 % der Haare in der telogenen Phase vorliegt. Eine weitere Ursache ist in einer Verminderung der Anzahl der Haarfollikel zu sehen, die bei einem Menschen mit normalem Haarwuchs ca. 500 Follikel pro Quadratcentimeter betragen und sich bei Haarwuchsstörungen entsprechend verringern.

Es befindet sich eine Vielzahl der verschiedensten Mittel im Handel, die sowohl extern, in Form von Kosmetika wie Haarwasser, Haarshampoos und ähnlichem angewendet werden, als auch Mittel zur innerlichen Anwendungen, d.h. oral verabreichte Präparate unterschiedlichster Zusammensetzung, die häufig unerwünschte Nebenwirkungen mit sich bringen.

Es ist bereits bekannt, dass die Verabreichung verschiedener Aminosäuren zur Stärkung des menschlichen Organismus beitragen und dass bestimmte Kombinationen verschiedener Aminosäuren zu einer Muskelstärkung, Gehirnaufbau, Stimulierung des Haarwuchses etc. führen. Insbesondere eine Mischung der Aminosäuren Leucin, Isoleucin und Valin wurde allein oder in Kombination mit zum Beispiel Fruktose und/oder Glukose als Haarwuchsmittel angeboten. Über die tatsächliche Wirkung dieses Präparats gibt es jedoch bei den im Rahmen der vorliegenden Erfindung getesteten Personen keinen eindeutigen Nachweis. Ein weiteres, zur rein kosmetischen Anwendung empfohlenes Präparat enthält neben nahezu allen 20 vorkommenden Aminosäuren die verschiedensten Vitamine, Nukleinsäuren, ATP, Calcium etc. Da keine nennenswerte Resorption von Nahrungsmitteln durch die Haut nachgewiesen werden kann, dürfte eine externe Anwendung ohne Nutzen sein. Dies gilt für Aminosäuren, Proteine, Fette und Fettsäuren. Eine externe Anwendung kann im allgemeinen die Qualität des bereits bestehenden Haares positiv beeinflussen, nicht jedoch dessen Wachstum. Wachstumsbedingte Schäden und Störungen können nur durch orale Einnahme wirkungsvoll bekämpft werden.

Ein weiteres Haarwuchsmittel ist aus der DE 36 37 992 bekannt, bei dem die Torulahefe in ihrer Gesamtheit als Haarwuchsmittelwirkstoff eingesetzt wird. Dabei wird lediglich festgestellt, dass die Vitamine und Aminosäuren an sich für den haarwuchsfördernden Effekt verantwortlich sind.

Der vorliegenden Erfindung liegt nun die Aufgabe zugrunde, ein physiologisches Präparat zu schaffen, das durch seinen speziellen Einfluss auf die verschiedenen Energiestoffwechselwege im menschlichen und tierischen Organismus sowie den Aufbau von Strukturproteinen einen positiven Einfluss auf den Haarwuchs und die Haarqualität entfaltet.

Diese Aufgabe wird durch ein Präparat mit der im kennzeichnenden Teil des Anspruchs 1 angegebenen Mischung gelöst.

Weitere vorteilhafte Ausgestaltungen der Erfindung sind in den abhängigen Ansprüchen angegeben.

Die Erfindung wird nachfolgend anhand von Beispielen und sechs Figuren näher erläutert.

Die Erfindung beruht auf der überraschenden Erkenntnis, dass die nichtessentielle Aminosäure Prolin, die sich durch ihre Ringstruktur mit geringem Raumbedarf auszeichnet, in Kombination mit den verzweigtkettigen essentiellen Aminosäuren Valin, Isoleucin und/oder Leucin, die Proteinbiosynthese der Hauptbestandteile des Haares, alpha-Keratin und Collagen, nachhaltig intensiviert und beschleunigt. Prolin nimmt eine Sonderstellung unter den Aminosäuren ein. Es ist in geringerem Masse als andere Aminosäuren an den sogenannten alpha-Helices der Proteine beteiligt, kommt aber sehr häufig in den haarnadelförmigen beta-Schleifen vor und ist daher als "Helixbrecher"von besonderer Bedeutung für die Proteinstruktur.

Die vorliegende Erfindung verwendet ein Gemisch der vier Aminosäuren Valin, Leucin, Isoleucin und Prolin. Die prozentuale Zusammensetzung variiert je nach Anwendungszweck und kann darüberhinaus in vorzüglicher Weise dem individuellen Bedarf des Patienten angepasst werden. Die vier Aminosäuren können allein verabreicht oder aber durch weitere Aminosäuren wie Cystein, Glycin, Lysin, Serin, Arginin, Methionin und Ornithin, Vitamine der B-Gruppe wie Biotin, des weiteren Taurin, Kreatin, Fruktose und Adenosin-Triphosphat ergänzt werden. Wird bei einer der Behandlung vorangestellten Analyse festgestellt, dass ein Mangel an den Spuren- oder Mengenelementen Calcium, Chrom, Eisen, Kalium, Kobalt, Kupfer, Magnesium, Mangan, Molybdän, Natrium, Nickel, Phosphor, Selen, Vanadium oder Zink besteht, können auch diese ergänzend zugeführt werden.

Durch die erfindungsgemässe Anwendung des Aminosäuregemisches bestehend aus Valin, Leucin, Isoleucin und Prolin, das gegebenenfalls durch weitere obenaufgeführte Aminosäuren, Vitamine und verschiedene Elemente ergänzt werden kann, kann es zudem zu einer deutlichen Verbesserung der verschiedenen Energiestoffwechselwege kommen, was sich zusätzlich positiv auf Leistungsfähigkeit, Muskelaufbau, Gehirnleistung, Fettabbau, Blutfett- und Blutzuckergehalt, Bindegewebsfestigung und sogar Potenzschwäche auswirkt. Das Präparat nach der vorliegenden Erfindung trägt in vorzüglicher Weise zu einer ausgeprägten Stimulierung des Haarwachstums bei, wie bereits weiter oben ausgeführt.

Das erfindungsgemässe Präparat kann in Form von Pulver, Kapseln, Tabletten oder in Flüssigkeit gelöst oral verabreicht werden. Das erfindungsgemässe Mittel kann ferner neben den wirksamen Bestandteilen die üblichen Hilfs-, Träger- und Zusatzstoffe enthalten. Es kann auch mit fruktose oder Zuckersyrup gebunden, getrocknet, zu Granulat verarbeitet verabreicht werden.

Die folgenden Beispiele stellen erfindungsgemäße Beispiele das, sofern deren Mengenangaben im Einklang unit den in Anspruch 1 definierten Mengenbereichen sind.

### Beispiel 1

Die folgende Zusammensetzung des erfindungsgemässen Präparats erwies sich als besonders wirkungsvoll:

| **Wirkstoffe** | **Anteile in %** | **Vorzugsweise** |
|---|---|---|
| **Cystein** | 0 - 10 % | 2 % |
| **Glycin** | 0 - 10 % | 2 % |
| **Isoleucin** | 5 - 25 % | 15 % |
| **Lysin** | 0 - 10 % | 2 % |
| **Leucin** | 10 - 40 % | 22 % |
| **Prolin** | 10 - 60 % | 40 % |
| **Valin** | 5 - 20 % | 11 % |
| | | |
| **ATP** | 0 - 10 % | 1,9 % |
| **Biotin** | 0 - 5 % | 0,1 % |

Zwölf Patienten mit Haarwuchstörungen wurde während 6 Monaten die Viererkombination Valin, Leucin, Isloleucin und Prolin täglich verabreicht. Bereits nach 3 Monaten war eine auffällige Verbesserung des Haarwachstums zu verzeichnen.

Einer Kontrollgruppe von 3 Patienten wurde die bereits bekannte Dreierkombination Valin, Leucin und Isoleucin verabreicht. Nach dreimonatiger Behandlung konnte keine Verbesserung der Haarwuchsstörung bei diesen Patienten verzeichnet werden, weshalb auch hier auf die neue Viererkombination übergegangen wurde. Nach dreimonatiger Behandlung der Kontrollgruppe mit der Viererkombination verbesserten sich auch bei diesen Patienten die Haarwuchsstörungen merklich, d.h. der Haarausfall pro Tag verringerte sich (siehe Figuren 1 und 2), die Haarwuchsphasen verschoben sich von der katagenen und telogenen Phase hin zur anagenen Phase (siehe Figuren 3 und 4) und auch die Struktur der Haarschäfte verbesserte sich merklich (siehe Figuren 5 und 6).

### Beispiel 2

Die folgende Zusammensetzung des erfindungsgemässen Präparats erwies sich als besonders wirkunsvoll:

| **Wirkstoffe** | **Anteile in %** | **Vorzugsweise** |
|---|---|---|
| **Arginin** | 0 - 30 % | 10 % |
| **Carnitin** | 0 - 10 % | 5 % |
| **Cystein** | 0 - 10 % | 3 % |
| **Cystin** | 0 - 10 % | 1 % |
| **Glycin** | 0 - 20 % | 8 % |
| **Isoleucin** | 5 - 20 % | 10 % |
| **Kreatin** | 0 - 10 % | 4 % |
| **Leucin** | 10 - 40 % | 15 % |
| **Lysin** | 0 - 20 % | 5 % |
| **Methionin** | 0 - 10 % | 2 % |
| **Ornithin** | 0 - 30 % | 10 % |
| **Prolin** | 5 - 30 % | 10 % |
| **Serin** | 0 - 5 % | 1 % |
| **Taurin** | 0 - 15 % | 5 % |
| **Valin** | 0 - 20 % | 8 % |
| | | |
| **ATP** | 0 - 5 % | 2 % |
| **Biotin** | 0 - 1 % | 0,25 % |
| **Fructose** | 0 - 5 % | 0,75 % |

### Beispiel 3

Die im folgenden aufgeführte Zusammensetzung des erfindungsgemässen Präparats bezieht sich auf ein sogenanntes Basispräparat und eine Ergänzung:

| **Basis:** | | |
|---|---|---|
| **Wirkstoffe** | **Anteile in %** | **Beispielsweise** |
| **Cystein** | 0 - 5% | 3% |
| **Glycin** | 0 - 10% | 3,5% |
| **Isoleucin** | 5 - 25% | 20% |
| **Leucin** | 10 - 40% | 30% |
| **Methionin** | 0 - 10% | 2% |
| **Prolin** | 5 - 75% | 10% |
| **Taurin** | 0 - 10% | 2,5% |
| **Valin** | 10 - 20% | 15% |

| **Ergänzungen zur Basis** | | |
|---|---|---|
| **Wirkstoffe** | **Anteile in %** | **Beispielsweise** |
| **Arginin** | 0 - 10 % | 2 % |
| **Carnitin** | 0 - 5 % | 1,25 % |
| **Kreatin** | 0 - 5 % | 1,25 % |
| **Cystin** | 0 - 5 % | 1,25 % |
| **Lysin** | 0 - 10 % | 3 % |
| **Ornithin** | 0 - 5 % | 1,25 % |
| **Serin** | 0 - 5 % | 1,25 % |
| | | |
| **ATP** | 0 - 5 % | 1,25 % |
| **Biotin** | 0 - 1 % | 0,25 % |
| **Fructose** | 0 - 5 % | 1,25 % |

Während sich die Zusammensetzung nach Beispiel 1 als besonders wirkungsvoll zur Behandlung von Haarwuchsstörungen erwies, erwies sich die Zusammensetzung nach Beispiel 2 als besonders wirkungsvoll bei der Unterstützung der verschiedenen Energiestoffwechsel dahingehend, dass eine positive Wirkung bezüglich Leistungsfähigkeit, Muskelaufbau, Gehirnleistung, Fettabbau, Blutfettgehalt, Blutzuckergehalt, Haut- und Haarregeneration, Bindegewebsfestigung, Müdigkeit und Potenzschwäche erreicht werden kann.

### Beispiel 4

Die folgende Zusammensetzung des erfindungsgemässen Präparats erwies sich auch als besonders wirkunsvoll:

| **Wirkstoffe** | **Anteile in %** | **Beispielsweise** |
|---|---|---|
| **Cystein** | 0 - 25 % | 5 % |
| **Glycin** | 0 - 35 % | 5 % |
| **Isoleucin** | 0 - 35 % | 20 % |
| **Lysin** | 0 - 25 % | 5 % |
| **Leucin** | 0 - 50 % | 30 % |
| **Prolin** | 4 - 100 % | 15 % |
| **Valin** | 0 - 30 % | 15 % |
| | | |
| | | % |
| **ATP** | 0 - 20 % | 4,75 % |
| **Biotin** | 0 - 5 % | 0,25 % |

### Beispiel 5

Die im folgenden aufgeführte Zusammensetzung des erfindungsgemässen Präparats bezieht sich auf ein sogenanntes Basispräparat, eine Hauptergänzung und eine weitere Ergänzung:

| **Basis:** | | |
|---|---|---|
| **Wirkstoffe** | **Anteile in %** | **Beispielsweise** |
| **Isoleucin** | 5 - 35 % | 18 % |
| **Leucin** | 10 - 50 % | 27 % |
| **Prolin** | 10 - 80 % | 15 % |
| **Valin** | 5 - 30 % | 14 % |

| **Hauptergänzungen zur Basis** | | |
|---|---|---|
| **Wirkstoffe** | **Anteile in %** | **Beispielsweise** |
| **ATP** | 0 - 20 % | 4 % |
| **Biotin** | 0 - 5 % | 1/4 % |
| **Cystein** | 0 - 20 % | 5 % |
| **Glycin** | 0 - 30 % | 5 % |
| **Lysin** | 0 - 20 % | 5 % |

| **Weitere Ergänzungen zur Basis** | | |
|---|---|---|
| **Wirkstoffe** | **Anteile in %** | **Beispielsweise** |
| **Arginin** | 0 - 10 % | 0,50 % |
| **Carnitin** | 0 - 10 % | 0,50 % |
| **Cystin** | 0 - 15 % | 1,00 % |
| **Kreatin** | 0 - 10 % | 0,50 % |
| **Methionin** | 0 - 15 % | 1,50 % |
| **Ornithin** | 0 - 10 % | 0,50 % |
| **Serin** | 0 - 15 % | 1,25 % |
| **Taurin** | 0 - 15 % | 1,00 % |

Die vorliegende Erfindung umfasst demzufolge ein diäthetisches Präparat zur Aufrechterhaltung beziehungsweise Regelung der verschiedenen Energiestoffwechselwege im menschlichen und tierischen Organismus, zur Intensivierung und Beschleunigung der Proteinbiosynthese insbesondere für die Hauptbestandteile der Haare, α-Keratin und Collagen, wobei eine wirksame Menge eines Gemisches aus den vier Aminosäuren Valin, Isoleucin, Leucin und Prolin oral verabreicht wird.

Das Basispräparat nach dem Beispiel 3 oder 5 kann auch als Grundlage der in den Beispielen 1, 2 oder 4 aufgeführten Zusammensetzungen dienen und darüberhinaus je nach individuellem Bedürfnis des Patienten sowie nach den zu behandelnden Symptomen abgeändert werden.

Ergibt eine Analyse entweder des Haares oder des Blutes eines Patienten einen Mangel an Spurenelementen, so können die im folgenden aufgeführten Elemente den unter den Beispielen 1 bis 5 sowie dem hier beschriebenen Basispräparat aufgeführten Zusammensetzungen ebenfalls beigemischt werden:

| | | | |
|---|---|---|---|
| Kalium | (I-wertig) | Natrium | (I-wertig) |
| Calcium | (II-wertig) | Eisen | (II-wertig) |
| Kobalt | (II-wertig) | Kupfer | (II-wertig) |
| Magnesium | (II-wertig) | Mangan | (II-wertig) |
| Molybdän | (II-wertig) | Nickel | (II-wertig) |
| Selen | (II-wertig) | Vanadium | (II-wertig) |
| Zink | (II-wertig) | Chrom | (III-wertig) |
| Phosphor | (diverse Wertigkeiten) | | |

Vorzugsweise wird zur Verbesserung des qualitativen und quantitativen Haarwuchses, der Hautstruktur und/oder der Nagelregeneration eine Mischung der Aminosäuren Valin zu Leucin zu Isoleucin ein Verhältnis 7 ± 20% : 17 ± 20% : 11 ± 20% verwendet, wobei die Mischung zusätzlich Prolin enthält.

Diese vier Aminosäuren können durch Zugabe weiterer Aminosäuren, Vitamine der B-Gruppe, Enzyme, Fruktose, Adenosin-Triphosphat und Spurenelemente ergänzt werden. Das erfindungsgemässe Präparat normalisiert den Haarausfall, verbessert die Haarqualität, aktiviert ruhende Haarwurzeln und fördert damit den quantitativen Haarwuchs nachhaltig. Ausserdem kann es sich positiv auf Leistungsfähigkeit, Muskelaufbau, Gehirnleistung, Fettabbau, Blutfettgehalt, Blutzuckergehalt, Bindegewebsfestigung und Potenzschwäche auswirken.

Das Präparat zur Verbesserung des Haarwuchses, der Hautstruktur und/oder der Nagelregeneration kann auch eine Mischung von nur Prolin und Trägerstoffen umfassen, und zwar derart, dass es zu einer bekömmlichen oralen Verabreichung präpariert ist. Bei einem solchen Präparat können die Trägerstoffe beispielsweise ein Granulat mit Zuckersirup, oder Schokolade oder schokoladenartige Produkte sein. Das Präparat kann auch Waffeln umfassen, auf oder zwischen oder in denen Prolin oder eine Prolinmischung aufgetragen ist, wobei vorzugsweise das ganze Präparat eine Schokoladenummantelung aufweist. Die Waffeln können auch rohrförmig und mit dem Prolin oder der Prolinmischung gefüllt sein.

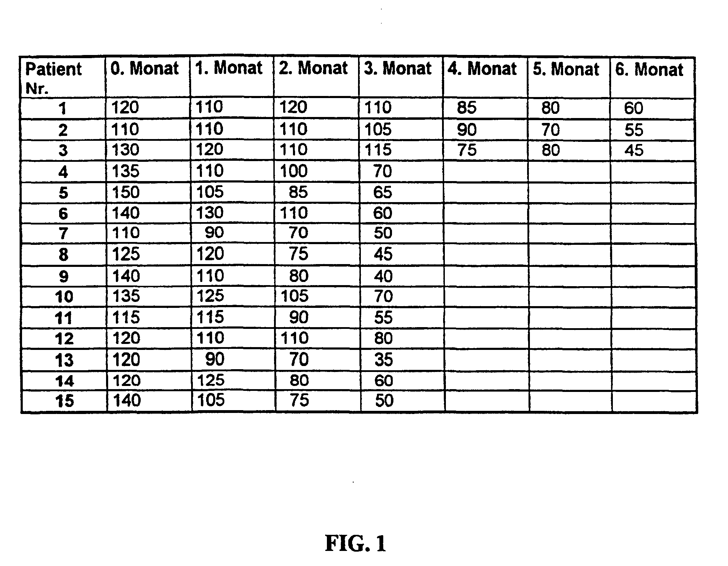

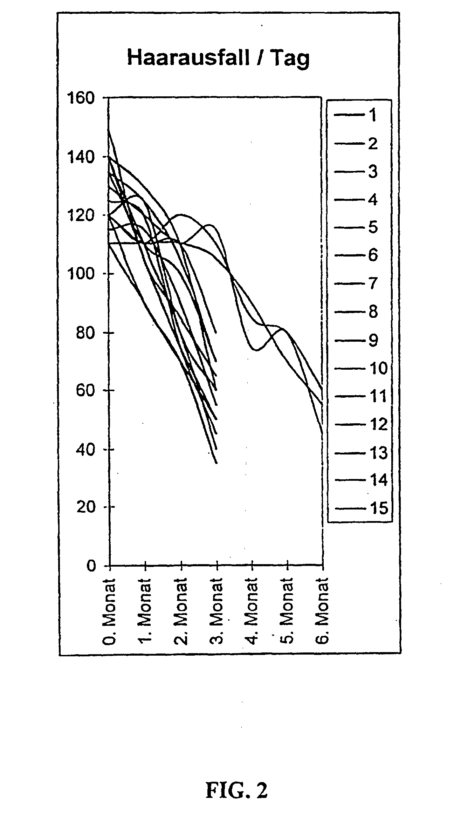

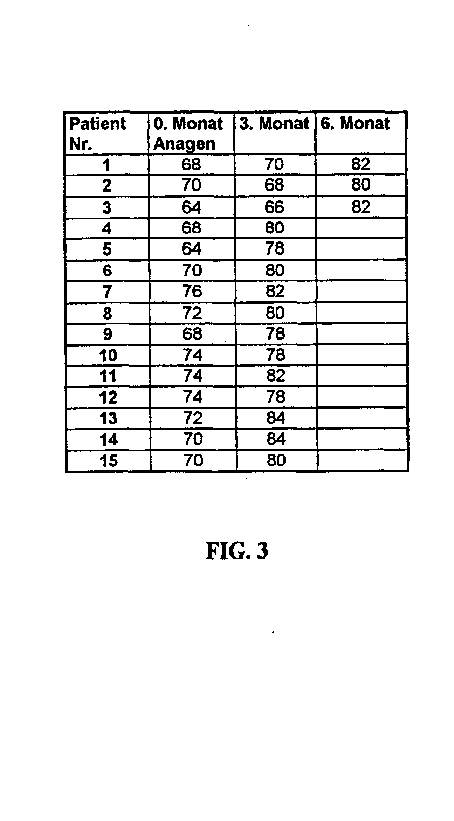

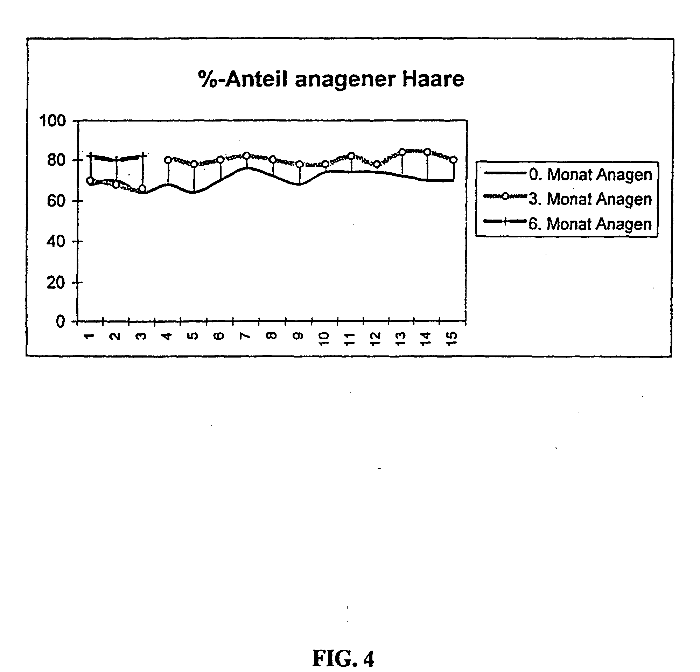

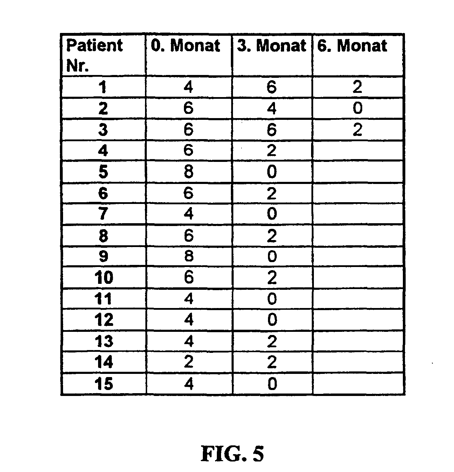

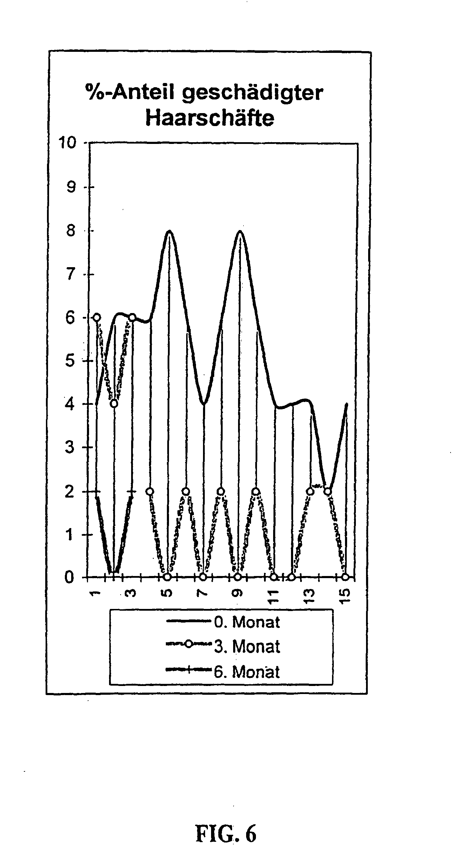

Die Figuren 1 und 2 zeigen eine Tabelle und ein Diagramm über den Haarausfall (Anzahl Haare pro Tag) von 15 Patienten, die Figuren 3 und 4 die Haarzyklus-Phase anagen in % (zusammen mit Katagen und Telogen = 100 %) und die Figuren 5 und 6 die Struktur des Haarschaftes (Anzahl geschädigter Haarschäfte in %). Die Haarzyklusphasen wurden durch mikroskopische Analyse von min. 50 extrahierten Haaren (Trichogramm) unterschieden (unter normalen Bedingungen: Anagenphase = 80 bis 85 %).

## Patentansprüche

1. Präparat zur Verbesserung des Haarwuchses und/oder der Nagelregeneration, **dadurch gekennzeichnet, dass** das Präparat eine Mischung der Aminosäuren Valin, Leucin und Isoleucin im Verhältnis von 7:17:11 plus/minus 20% umfasst, wobei Valin zu 5-30 Gewichtsprozent, Leucin zu 10-50 Gewichtsprozent und Isoleucin zu 5-35 Gewichtsprozent enthalten ist und die Mischung weiterhin 10-80 Gewichtsprozent Prolin und Trägerstoffe umfasst und zur oralen Verabreichung präpariert ist, wobei die genannte Mischung auf individuelle Bedürfnisse beziehungsweise Symptome von Patienten beziehungsweise Patientengruppen angepasst wird.

2. Präparat nach Anspruch 1, **dadurch gekennzeichnet, dass** mehr als 5 Gew.% Valin, mehr als 10 Gew.% Leucin, mehr als 5 Gew.% Isoleucin und mehr als 15 Gew.% Prolin enthalten sind.

3. Präparat nach den Ansprüchen 1 und/oder 2, **dadurch gekennzeichnet, dass** es 10-30 Gew.% Valin, 10-40 Gew.% Leucin, 15-35 Gew.% Isoleucin und 10 Gew.% Prolin umfasst.

4. Präparat nach Anspruch 1, **dadurch gekennzeichnet, dass** es 22 Gew.% Valin, 44 Gew.% Leucin, 30 Gew.% Isoleucin und 10 Gew.% Prolin umfasst.

5. Präparat nach einem oder mehreren der Ansprüche 1 bis 4, **dadurch gekennzeichnet, dass** es je nach individuellem Bedarf der Patientin/des Patienten 0-10 Gew.% Cystein und/oder 0-15 Gew.% Cystin und/oder 0-10 Gew.% Glycin und/oder 0-10 Gew.% Lysin und/oder 0-15 Gew.% Methionin und/oder 0-10 Gew.% ATP und/oder 0-5 Gew.% Biotin umfasst.

6. Präparat nach einem oder mehreren der Ansprüche 1 bis 5, **dadurch gekennzeichnet, dass** eines oder mehrere der Spurenelemente Calcium, Chrom, Eisen, Kalium, Kobalt, Kupfer, Magnesium, Mangan, Molybdän, Natrium, Nickel, Phosphor, Selen, Vanadium und Zink zugegeben werden.

7. Präparat nach einem oder mehreren der Ansprüche 1 bis 6, **dadurch gekennzeichnet, dass** es zur oralen Präskription in Pulver-, Tabletten-, Kapselform oder als lösliches Mittel zur Verabreichung in Flüssigkeit, jedoch vorzugsweise als Granulat präpariert ist.

8. Präparat nach einem oder mehreren der Ansprüche 1 bis 7, **dadurch gekennzeichnet, dass** es die üblichen Hilfs-, Träger- und Zusatzstoffe umfasst.

9. Präparat nach einem oder mehreren der Ansprüche 1 bis 8, **dadurch gekennzeichnet, dass** die Trägerstoffe ein Granulat mit Zucker- und/oder Fructosesirup umfassen.

## Claims

1. A preparation for the improvement of the hair growth and/or nail regeneration, **characterized in that** the preparation comprises a mixture of the amino acids valine to leucine to isoleucine of 7:17:11 plus/minus 20%, wherein the preparation contains 5 - 30 wt.% valine, 10 - 50 wt.% leucine, 5 - 35 wt.% isoleucine and that the mixture contains 10 - 80 wt.% proline and carrier substances and is prepared for oral administration, wherein said mixture is conditioned for the individual needs or symptoms, respectively, of patients or group of patients, respectively.

2. The preparation according to claim 1, **characterized in that** it contains more than 5 wt.% valine, more than 10 wt.% leucine, more than 5 wt.% isoleucine and more than 15 wt.% proline.

3. The preparation according to claim 1 and/or 2, **characterized in that** it contains 10 - 30 wt.% valine, 10 - 40 wt.% leucine, 15 - 35 wt.% isoleucine and 10 wt.% proline.

4. The preparation according to claim 1, **characterized in that** it contains 22 wt.% valine, 44 wt.% leucine, 30 wt.% isoleucine and 10 wt.% proline.

5. The preparation according to one of claims 1 to 4, **characterized in that** it contains 0 - 10 wt.% cysteine and/or 0 - 15 wt.% cystine and/or 0 - 10 wt.% glycine and/or 0 - 10 wt.% lysine and/or 0 - 15 wt.% methionine and/or 0 - 10 wt.% ATP and/or 0 - 5 wt.% biotine, depending on the individual needs of the male or female patient.

6. The preparation according to one of claims 1 to 5, **characterized in that** one or several of the trace elements calcium, chrome, iron, potassium, cobalt, copper, magnesium, manganese, molybdenum, sodium, nickel, phosphorus, selenium, vanadium and zinc are added.

7. The preparation according to one of claims 1 to 6, **characterized in that** it is prepared for oral prescription in the form of powder, tablets, capsules or soluble preparation to be administered in a liquid, preferably, however, as granules.

8. The preparation according to one of claims 1 to 7, **characterized in that** it contains the usual auxiliary agents, carriers and additives.

9. The preparation according to one of claims 1 to 8, **characterized in that** the carriers comprise granules with sugar and/or fructose syrup.

## Revendications

1. Une préparation pour améliorer la pousse des cheveux et/ou régénérer les ongles, **caractérisée en ce que** la préparation comprend une mixture des aminoacides, valine, leucine et isoleucine de 7:17:11 plus ou moins 20%, où la préparation comprend 5 - 30 % en poids de valine, 10 - 50 % en poids de leucine, 5 - 35 % en poids de isoleucine et **en ce que** la mixture comprend 10 - 80 % en poids de proline et des substances porteurs et est préparée pour une administration orale, où ladite mixture est conditionnée pour des besoins individuels ou des symptômes de patients ou de groupes de patients.

2. Préparation suivant la revendication 1, **caractérisée en ce que** elle comprend plus que 5 % en poids de valine, plus que 10 % en poids de leucine, plus que 5 % en poids de isoleucine et plus que 15 % en poids de proline.

3. Préparation suivant la revendication 1 et/ou 2, **caractérisée en ce que** elle comprend 10 - 30 % en poids de valine, 10-40 % en poids de leucine, 15 - 35 % en poids de isoleucine et 10 % en poids de proline.

4. Préparation suivant la revendication 1, **caractérisée en ce que** elle comprend 22 % en poids de valine, 44 % en poids de leucine, 30 % en poids de isoleucine et 10 % en poids de proline.

5. Préparation suivant une des revendications 1 à 4, **caractérisée en ce que** elle comprend 0 - 10 % en poids de cystéine et/ou 0 - 15 % en poids de cystine et/ou 0 - 10 % en poids de glycine et/ou 0 - 10 % en poids de lysine et/ou 0 - 15 % en poids de méthionine et/ou 0 - 10 % en poids de ATP et/ou 0 - 5 % en poids de biotine suivant les besoins individuels d'un patient ou d'une patiente.

6. Préparation suivant une des revendications 1 à 5, **caractérisée en ce que** un ou plusieurs des oligo-éléments calcium, chrome, fer, potassium, cobalt, cuivre, magnésium, manganèse, molybdène, sodium, nickel, phosphore, sélénium, vanadium et zinc sont additionnés.

7. Préparation suivant une des revendications 1 à 6, **caractérisée en ce que** elle est préparée pour une prescription orale dans la forme d'une poudre, des tablettes, des capsules ou en tant que préparation soluble pour une administration dans un liquide, mais préférablement en tant que granulés.

8. Préparation suivant une des revendications 1 à 7, **caractérisée en ce que** elle comprend les agents auxiliaires, porteurs et additives usuels.

9. Préparation suivant une des revendications 1 à 8, **caractérisée en ce que** les agents auxiliaires comprennent des granulés avec un sirop de sucre et/ou de fructose.
